# EUROPÄISCHE PATENTANMELDUNG

(11) **EP 0 735 135 A2**
(43) Veröffentlichungstag der Anmeldung: **02.10.1996**
(21) Anmeldenummer: 96250073.2
(22) Anmeldetag: 29.03.1996
(51) Int. Cl.: C12N 5/08, C12M 1/00

(54) **Zellschichten und Transportsystem für Zellschichten**

(30) Priorität: 31.03.1995 DE 19513177; 22.03.1996 DE 19512998; 22.03.1996 DE 29620965 U
(71) Anmelder: DIZG Deutsches Institüt für Zell- und Gewebeersatz gGmbH, 13125 Berlin (DE)
(72) Erfinder: Wilke, Barbara, Dr., D-17309 Jatznick (DE); von Versen, Rüdiger, Dr., D-16352 Basdorf (DE); Rakow, Adalbert, Dr., D-13187 Berlin (DE); Mönig, Hans-Joachim,, D-12557 Berlin (DE)
(74) Vertreter: Wehlan, Helmut, Dr.

(57) **Zusammenfassung**

Die Erfindung betrifft gezüchtete Zellschichten und Amnionpräparate, Verfahren zu ihrer Herstellung, ihre Verwendung in der Humanmedizin und ein Transportsystem für Zellschichten und Amnionpräparate, wie Haut-Transplantationsgewebe, die unter definierten Bedingungen vom Gewinnungs- zum Einsatzort gelangen müssen. Die Substrat-Zellschichten enthalten neue kollagenhaltige oder gelartige Träger sowie Amnion oder azelluläre Dermis als Substrat und eine Schicht aus gezüchteten Zellen, wie Keratinozyten, Chondrozyten, Fibroblasten oder Endothelzellen.

## Beschreibung

Die Erfindung betrifft gezüchtete Zellschichten und Amnionpräparate, Verfahren zu ihrer Herstellung, ihre Verwendung in der Humanmedizin und ein Transportsystem für Zellschichten und Amnionpräparate, wie Haut-Transplantationsgewebe, die unter definierten Bedingungen vom Gewinnungs- zum Einsatzort gelangen müssen.

Seitdem Bell und Mitarbeiter partiell in vitro rekonstruierte Gewebe der Lederhaut gewonnen hatten (Bell et al., Proc. Natl. Acad. Sci. 76 (1979) 1274), sind die Verfahren zur Gewinnung von Hautgewebe ständig weiterentwickelt worden. Zuerst hatten Green und Mitarbeiter auch die Züchtung von Keratinocyten über einen längeren Zeitraum beschrieben (Green, H. et al., Proc. Natl. Acad.Sci. 76 (1979) 5665): Die Epidermisschicht entwickelte sich unter Bildung eines drei bis fünf Zellschichten dicken Gewebes sehr schnell. Diese Gewebe, bei Patienten transplantiert, differenzierten sich in situ weiter. Patienten mit schweren Verbrennungen konnten mit diesem Verfahren gerettet werden (G. Gallico et al., New England J. Med., 311 (1984) 448). Mit dem Green-Verfahren (US-(PS - Patentschritt) 4456687) kann aus einer Biopsie von 2 cm² innerhalb von drei bis vier Wochen bis zu einem Quadratmeter Epidermis gewonnen werden.

Embryonalhüllen, auch Eihäute, Fruchthüllen oder Keimhüllen genannt, stellen vom tierischen und menschlichen Embryo stammende Zellverbände dar, die den Körper umhüllen. Sie reißen vor der oder bei der Geburt auf. Die Amnionhüllen bestehen aus der inneren (Amnion) und aus der äußeren (Chorion) Hülle. Die Amnionhöhle ist mit Fruchtwasser zum weitgehenden Schutz des Embryos gefüllt (Lexikon der Biologie, Spektrum Akademischer Verlag Heidelberg - Berlin - Oxford, 1994).

Amnien werden seit vielen Jahren als Wundabdeckungsmaterialien eingesetzt, insbesondere als zeitweiliger Hautersatz bei Verbrennungswunden, insbesondere Human-Amnion-Membranen (z.B. Kucan, J.O. et al., Ann.-Plast-Surg. 1982, 8 (6): 523-527; Lin, SiD. et al., Burns-Incl-Therm-Inj. 1985, 11 (5): 374-378; Gobel, P. et al., Beitr-Orthop-Traumatol. 1990, 37 (9): 495-498), aber auch in der allgemeinen Chirugie, z.B. als Wundverband bei chronischen Ulzerationen (Shun, A. et al., Med-J-Aust. 1983, 2 (6): 279-283), in der Orthopädie zur Vermeidung von Infektionen (Vishwakarma, G.K. et al., J-Bone-Joint-Surg-Br. 1986, 68 (1): 68-74) oder in der rekonstruktiven Chirugie (Lawson,V.G., Aust-N-Z-Y-Surg. 1986, 56 (2): 163-166; Zohar, Y. et al., Laryngoscope, 1987, 97 (8 Pt 1): 978-980; Fishman, I.J.et al., J-Urol. 1987, 138 (5): 1291-4).

Die Verwendung der menschlichen Plazenta als Ausgangsprodukt für die Gewinnung von Implantaten für die Gefäß- und Hautchirurgie ist Gegenstand der DE-OS (Deutsche Offenlegungsschrift) 3608329 (Bunge, J., 1987). Darin wird auch das Amnion als geeignetes Hauttransplantat zum Decken von großflächigen Verbrennungen beschrieben (vgl. auch DE-OS 3240909, Fauck, W.P., 1983). Amnion-Membranen sind auch als Substratgewebe zur Bewertung des Wachstums von Zellen sowie als Wachstumssubstrat für Zellkulturen beschrieben worden (US-PS (Patentschrift) 4446234, Russo, R. et al. 1984) beschrieben worden.

Es ist ferner bekannt, daß eine bevorzugte Methode zur Aufbewahrung von Hauttransplantaten, z.B. in der Europäischen Haut-Bank, darin besteht, Spender-Haut in Glycerol zu konservieren (de Backere, A.C.J. : "EURO Skin Bank: large scale skin-banking in Europe based on glycerol-preservation of doner skin", Burns 1994 20 (1) 4-9; Hettich R. et al. : "The immunogenicity of glycerol-preserved doner skin", Burn 1994, 20 (1) 71-76. Kürzlich ist auch ein neues Protein, Kalinin genannt, bekanntgeworden, das die Adhäsion transplantierter Keratinozyten auf eine Substratunterlage, u.a. auch Amnion, verbessern soll (US-PS 5352668, Burgeson, R.E., 1994).

Die Herstellung des Gewebes und sein Transport zum Einsatzort ist mit einer Reihe technischer Probleme verbunden. Einen besonderen Raum nehmen dabei die Trägermaterialien ein, die einen Transport des Gewebes und die Transplantation auf den Patienten ermöglichen müssen.

Deshalb sind vielfältige Trägermaterialien vorgeschlagen worden, auf biologischer und auf synthetischer Grundlage. Gemäß DD 297678 wird ein biologisches Trägermaterial beschrieben, das aus Mischtransplantaten von Plasmaproteinkonzentrat und Calciumthrombin besteht.

Andere Vorschläge bestehen in der Verwendung von Bindegewebszellen (DE 3829183), Mischtransplanten aus Eigenhaut und Leichenhaut (DE 35392709), Kadaver-Allotransplantaten, Xeno-Transplantaten von Schweinehaut, künstlicher Haut, Kollagenen oder Kunststoffen, wie thermoplastischen Copolyestern (DE 2651685 ,3716907, 3711699, 4127570; WO 92/06179, 91/16010; US 5282859, 5147401, 4888291, 4883487, 4642118).

Der Lebend-Lagerfähigkeit und der Aufrechterhaltung der koloniebildenden Effektivität von Epithelzellschichten widmet sich die EP 507849 (US 5100676, WO 91/11100, DE 69100748).

Darin wird die Aufbewahrung der Zellschicht (z.B. Keratinocyten) in einem physiologisch verträglichen Medium, das Aufbringen der kohäsiven Schicht auf ein nicht anhaftendes Substrat, die Verpackung als Verband für Hautwunden und die Aufbewahrung als Kühlpackung für einen Zeitraum bis zu 26 Stunden beschrieben.

Der Erfindung liegt die Aufgabe zugrunde, neue Zellschichten, Verfahren zu ihrer Herstellung, ihre Verwendung in der Humanmedizin und ein Transportsystem für Zellschichten zu entwickeln. Eine weitere Aufgabe besteht darin, Amnionpräparate zur Verfügung zu stellen, die sich in einem gebrauchsfähigen Zustand zur Vorratshaltung eignen. Die Aufgaben wurden dadurch gelöst, daß biologische und / oder nichtbiologische Trägermaterialien als nicht anhaftende Substrate zur Bildung von Zellschichten eingesetzt werden, insbesondere neue kollagenhaltige oder gelartige Träger sowie Amnion oder azelluläre Dermis. Auf diese Träger werden die zur Schichtbildung vorgesehenen Zellen gebracht und in einem Nährmedium gezüchtet. Als Zelltypen finden erfindungsgemäß humane Epithelzellen (Keratinozyten), Knorpelzellen (Chondrozyten), Fibroblasten oder Endothelzellen Anwendung. Geeignete Nährmedien sind vor allem solche Kulturlösungen, die Aminosäuren, Fettsäuren, Puffersalze, Spurenelemente und Vitamine enthalten, ebenso bekannte Nährmedien, wie MEM oder Ham F 12, aber auch das Zellkulturmedium MCDB 153.

Im Fall der Amnionpräparate wurde die Aufgabe dadurch gelöst, daß Embryonalhüllen von der Plazenta abgetrennt, von anhaftenden Bestandteilen befreit, in feuchtem Zustand das Chorion abgestreift und die verbleibenden Amnien in konservierenden Lösungen aufbewahrt werden. Es finden insbesondere Embryonalhüllen nach Spontangeburten Verwendung. Humane Amnionpräparate setzen Spenderinnen voraus, die ihre Zustimmung zu einer Befragung über mögliche Ausschlußkriterien und zu infektionsserologischen Untersuchungen erteilt haben. In diesem Fall werden die Embryonalhüllen tiefgefroren und bis zur Freigabe durch negative infektionsserologische Befunde zwischengelagert. Das Abstreifen des Chorions erfolgt auf einer desinfizierbaren festen Untertage. Nach der Sterilisierung der zugeschnittenen Amnien werden die Präparate in konservierenden Agenzien unter Kühlung oder nach Gefriertrocknung aufbewahrt. Es hat sich herausgestellt, daß die erfindungsgemäßen Amnionpräparate gute hautverträgliche Eigenschaften zeigen und als Trägermaterial für Zelltypen, die im Laufe des Zellwachstums mehrschichtige Lagen bilden, wie Keratinozyten, geeignet sind.

Die Aufgabe, ein Transportsystem für Zellschichten zu entwickeln, wurde durch einen Transportbehälter gelöst, der aus einer bruchsicheren Außenverkleidung und einem Innenraum besteht, der lose Zwischenböden zur Lagerung der einzelnen, mit Nährmedium gefüllten und die Zellschichten enthaltenden Transportgefäße - wobei die Zwischenböden durch Stempel fixiert werden - und Heizquellen zur Temperaturregelung sowie eine steuerbare CO₂-Zufuhr enthält.

Die Außenverkleidung besteht vorzugsweise aus rostfreiem Stahlblech oder aus Edelmetall, die Isolierung des Behälters aus Kunststoff, vorzugsweise aus Polystyrol. Als Heizquellen werden Batterien bzw. aufladbare Akkumulatoren eingesetzt. Dieses Transportsystem, eine Kombination aus bekannten und aus neuen Teilen, eignet sich für bekannte wie für die erfindungsgemäßen Zellschichten, die aus einer Substrat-Zellschicht-Einheit bestehen, wobei als Substrat neue kollagenhaltige oder gelartige Träger sowie Amnion oder azelluläre Dermis und als zur Schichtbildung vorgesehene Zelltypen Epithelzellen (Keratinozyten), Knorpelzellen (Chondrozyten), Fibroblasten, Endothelzellen oder tierische Zellen Anwendung finden. Es hat sich überraschenderweise herausgestellt, daß die mit dem erfindungsgemäßen Transportsystem möglichen Transportbedingungen ein Anwachsen der Zellschichten und die Koloniebildung der Zellen auf der Wunde in vivo nicht beeintächtigen. Sie ermöglichen lange Transportzeiten und das Einhalten der gewünschten Innentemperatur von 5 bis 40 ° C und für z.B. Epithelzellen aus Keratinozyten von 20 bis 37°C. Das Transportsystem ist zum Transport biologischer Materialien, die lebende Zellen enthalten, geeignet - z.B. patienteneigenen Materials für autologe, von Fremdmaterialien für allogene Transplantationen wie auch xenogener Materialien.

Die Erfindung soll anhand von Ausführungsbeispielen näher erläutert werden.

### Ausführungsbeispiele

**Beispiel 1: Zellschichten**
Auf einer Substratschicht in einem Nährmedium wird unter üblichen Bedingungen ein die spätere Zellschicht bildender Zelltyp kultiviert und durch Vermehrung zur Substrat-Zellschicht gebracht. Durch die Kultivierung verschiedener Zelltypen auf einer Substratschicht werden in definierten Nährmedien durch Stimulation der Zellproliferation klinisch und diagnostisch anwendbare und transportable, vitale, geschlossene Zellverbände hergestellt.
Beispiel 1.1
   Gem. Beispiel 1 werden auf kollagenbeschichteten Membranen in der Zellkultur unter wachstumsstimulierenden Bedingungen Keratinozyten zu Keratinozyten-Zellschichten gezüchtet.
Beispiel 1.2:
   Gem. Beispiel 1 und 1.1 wird aus azellulärer Dermis als Substrat und Keratinozyten ein Dermis-Keratinozyten-Präparat gebildet.
Beispiele 1.3 ff.
   gem. Beispiel 1 bis 1.2 werden gebildet:
   eine kollagenbeschichtete Trägermembran-Chondrozyten-Zellschicht, kollagenbeschichtete Membran-Fibroblasten-Zellschicht, kollagenbeschichtete-Endothel-Zellschicht, Azellulärdermis-Chondrozyten-Zellschicht, Azellulärdermis-Fibrobasten-Zellschicht, Azellulärdermis-Endothel-Zellschicht sowie Gel-Zellschichten: Gel-Keratinozyten-Zellschicht, Gel-Chondrozyten-Zellschicht, Gel-Fibroblasten-Zellschicht oder Gel-Endothel-Zellschicht.

**Beispiel 2: Amnionpräparate**

Beispiel 2.1: Allgemeine Vorschrift
Die Anlieferung der Embryonalhüllen erfolgt in gefrorenem bzw. gekühltem Zustand mit der Serumprobe in Beuteln oder anderen Behältern. Dabei beträgt die Aufbewahrungstemperatur bis zur Verarbeitung +4°C bis -80°C. Zur Verarbeitung wird das Material in einem geeigneten Gefäß in kaltem bis handwarmem Wasser aufgetaut. Nach dem Auftauen wird ein Wasserwechsel vorgenommen.
In diesem Wasserbad wird das Material in Gläsern mit Schraubverschluß geschüttelt, (etwa 3 bis 5 min). Dabei erfolgt das Lösen von Blut und anderen anhängenden Gewebebestandteilen sowie die teilweise Trennung von Amnion und Chorion.
Dann wird das Material auf eine glatte Präparierunterlage (desinfizierbar) gebracht und durch stumpfes Lösen Amnion vom Chorion getrennt (Chorion wird evtl. verworfen).
Nach Begradigen der Einrißstellen (um ein weiteres Einreißen zu verhindern) wird die Fläche vermessen und gekennzeichnet. Das derart präparierte Amnion wird in ein Spezialgefäß für Amnion gegeben, das durch Spezialöffnungen für unterschiedliche Medien durchflutbar ist.
Nach anschließendem Spülen unter fließendem Wasser erfolgt der Sterilisationsvorgang für 10 bis 240 Minuten unter Schütteln, wobei die Sterilisationslösung aus Glycerol, Peressigsäure (PES), Ethanol und Aqua dest. besteht und die PES-Konzentration zwischen 0,005 und 2 % beträgt.
Nach Ablauf der Sterilisationszeit erfolgen die weiteren Arbeitsgänge unter Reinraumbedingungen (Klasse A), wie z.B. Spülen in einem Gemisch von sterilisierter isotonischer Kochsalzlösung und sterilisiertem Glycerin.
Danach erfolgt die Entnahme aus dem o.g. Spezialspülgefäß und Qualitätskontrolle des Amnionpräparats, die Sterilprobenentnahme und die Konfektionierung (Endverpackung mit Überführung in sterilisiertes Glycerin und definitiven Verschluß).
Beispiel 2.2: Humanes Amnionpräparat

2.2.1. Entnahme
Die Entnahme der Embryonalhüllen von der Nachgeburt (Plazenta) geschieht auf der rechtlichen Basis der schriftlichen Zustimmung der Spenderin, der pharmazeutisch-relevanten Spenderausschlußkriterien und der schriftlichen Erklärung (Entnahmeprotokoll) des behandelnden Arztes zur Eignung des Gewebes für die Herstellung eines klinisch bedenkenlos einsetzbaren Präparates.
2.2.2. Zwischenlagerung und Transport
Die Zwischenkonservierung in der Klinik erfolgt
- in einer Flasche mit gesättigter Kochsalzlösung und Kühlschrankbedingungen oder
- in einem Plastbeutel verschlossen und gefroren bei mindestens -20°C oder darunter oder
- durch einfache Lagerung in physiologischer Kochsalzlösung und normothermer Lagerung oder Verschluß im Plastbeutel und normothermer Lagerung, wenn die Abholung innerhalb von 12 Stunden nach Entnahme erfolgt.

Der Transport wird in einer Isolierbox unter Kühlbedingungen vorgenommen. Innerhalb von 12 Stunden nach der Entnahme kann der Transport ohne zusätzliche Kühlung erfolgen. Eine Ausnahme sind Umgebungstemperaturen über 20°C.
2.2.3. Zwischenlagerung bis zur Präparation
Nach Anliefetung des Amnion-Ausgangsmaterials vom "Sammlungsort" (geburtshilfliche Einrichtungen) verbleibt das Gewebe in Quarantänelagerung ( -18°C oder darunter) bis zum Vorliegen der infektionsserologischen Befunde (AIDS, Hepatitis, Lues, Cytomegalie). Diese Zwischenlagerung wird bis zum Beginn der mechanischen Bearbeitung ausgedehnt.
2.2.4. Mechanische Präparation
In Vorbereitung auf die mechanische Präparation wird das Gewebe im Wasserbad aufgetaut. Es schließt sich ein Waschvorgang zur Entfernung von anhaftenden Geweben und von Blut an.
In diesem Wasserbad wird das Gewebe in Gläsern oder Plastflaschen mit Schraubverschluß etwa 3 bis 5 Minuten geschüttelt. Dabei erfolgt das erleichterte Lösen von Blut sowie die bereits teilweise Trennung von Amnion und Chorion.
Auf einer desinfizierbaren festen Unterlage wird dann das Amnion in feuchtem Zustand durch Abstreifen vom Chorion befreit.
Es schließt sich eine Begradigung eventueller Einrißstellen - mit Hilfe von Scheren oder anderen mechanischen Schneidinstrumenten - an. Damit verhindert man ein weiteres Einreißen und ein Unbrauchbarwerden des Präparates. Die verbleibende Fläche wird vermessen und das Präparat zur Kennzeichnung mit einer Kontrollmarke versehen.
2.2.5. Sterilisation
Das so behandelte und gereinigte Amnion wird nun einem Sterilisationsprozeß unterworfen. Diese Sterilisation erfolgt mit einem Peressigsäure-Alkoholgemisch. Dazu wird ein Spezialgefäß verwedet, das sowohl die Vermischung der einzelnen Amnienpräparate verhindert als auch durch speziell angebrachte Öffnungen leicht mit den verwendeten Lösungen durchflutet (durchströmt) werden kann.
Die Lösung besteht bevorzugt aus:

| | |
|---|---|
| - Glycerin | 375 ml |
| - Wofasteril | 19 ml |
| - Ethanol | 250 ml |
| - Aqua dest. | 356 ml. |

Den Sterilisationsvorgang führt man bevorzugt über einen Zeitraum von 2 Stunden unter Schütteln durch. Die Sterilisation wird durch einen Spülvorgang zum Auswaschen der Sterilisationslösung mit (bevorzugt) isotonischer steriler Kochsalzlösung und sterilem Glycerin durchgeführt. Nach Ablauf der Sterilisation erfolgen alle weiteren Arbeitsgänge unter Reinraumbedingungen (Reinraumklasse A).
2.2.6. Sterilkontrolle
Als nächster Schritt erfolgt die Entnahme des Amnions aus den o.g. Spezialgefäßen und die Entnahme einer Probe zur Sterilkontrolle.
2.2.7. Konfektionierung
Das sterilisierte Amnion wird nun in ein definitiv verschließbares Gefäß, bevorzugt aus Glas oder Kunststoff, welches mit sterilem Glycerin voll gefüllt ist, gebracht. Dies ist die Endverpackung.
2.2.8. Dokumentation, Label
nach Arzneimittelgesetz.
2.2.9. Endlagerung / Depothaltung
Bis zur Abgabe an den Besteller wird das Amnionpräparat bei Kühlschranktemperatur oder darunter gelagert.
Die Glycerinkonservierung gestattet auch eine schadensfreie Lagerung bei Minustemperaturen.
Dies bedeutet eine Verlängerung der Haltbarkeit und damit Verlängerung der Lagerungszeit.
Beispiel 2.2.10:
Gem. Beispiel 1 und 1.1 wird eine Amnion-Keratinozyten-Zellschicht gebildet.
Beispiel 2.2.11:
Gem. Beispiel 1 und 1.1 wird eine Amnion-Chondrozyten-Zellschicht gebildet.
Beispiel 2.2.12:
Gem. Beispiel 1 und 1.1 wird eine Amnion-Fibroblasten-Zellschicht gebildet.
Beispiel 2.2.13:
Gem. Beispiel 1 und 1.1 wird eine Amnion-Endothel-Zellschicht gebildet.
Beispiel 2.3: Bovines Amnionpräparat (Fließschema)
Die Embryonalhüllen werden nach Spontangeburten von der Plazenta abgetrennt, tiefgefroren und bei mindestens -18°C bis zur Freigabe durch negative infektionsserologische Befunde zwischengelagert.
Zur weiteren Aufarbeitung werden die Amnien im Wasserbad aufgetaut.
Zur Entfernung von Blut u.a. anhaftenden Bestandteilen werden die Embryonalhüllen mit Wasser gewaschen.
Auf einer desinfizierbaren festen Unterlage werden sie in feuchtem Zustand durch Abstreifen vom Chorion befreit. Das Chorion wird verworfen.
Das verbleibende Amnion wird nochmals mit Wasser gespült und anschließend auf die gewünschte Größe zugeschnitten.
Die Sterilisation erfolgt mit 1 %iger Peressigsäure unter Zusatz von Alkohol und Glycerin für 2 Stunden unter Schütteln.
Es folgt das Auswaschen der Sterilisationslösung mit isotonischer Kochsalzlösung und die Entnahme der Sterilitätskontrolle.
Es folgen: Konfektionieren des Präparates unter Laminar Air Flow (Reinraumklasse A) in definitiv verschließbaren Gefäßen in Glycerin.
Lagerung bei Kühlschranktemperatur oder darunter.
Abgabe des Präparates auf Anforderung.
Beispiel 2.4: Trägermaterial
Zelltypen, die im Laufe des Zellwachstums mehrschichtige Zellayer bilden, z.B. Keratinozyten, werden in Kulturgefäßen auf Feederzellen bzw. mit Basalmembranproteinen beschichteten Zellkulturoberflächen gezüchtet, die Zellayer enzymatisch abgelöst und auf verschiedene Trägermaterialien gebracht.
Die erfindungsgemäßen Amnionpräparate eignen sich in vorteilhafter Weise zur Verwendung solcher Trägermaterialien.
**Beispiel 3: Transportbehälter**
Figur 1
Bezugszeichen:
- 1: Außenverkleidung
- 2: Zwischenböden
- 3: Stempel
- 4: CO₂-Zufuhr
- 5: Heizquellen

Der Transportbehälter besteht aus einer bruchsicheren Außenverkleidung und einem Innenraum, der lose Zwischenböden zur Lagerung der Zellschichten enthält - die durch Stempel fixiert werden - und der, neben dem Nährmedium, Heizquellen zur Temperaturregelung und eine steuerbare CO₂-Zufuhr enthält. Die Außenverkleidung besteht vorzugsweise aus rostfreiem Stahlblech, die Isolierung des Behälters aus Kunststoff, vorzugsweise aus Polystyrol. Als Heizquellen werden Batterien bzw. aufladbare Akkumulatoren eingesetzt.

Dieses Transportsystem, eine Kombination aus bekannten und aus neuen Teilen, eignet sich für bekannte wie für die erfindungsgemäßen Zellschichten, die aus einer Substrat-Zellschicht-Einheit bestehen, wobei als Substrat neue kollagenhaltige oder gelartige Träger sowie Amnion oder azelluläre Dermis und als zur Schichtbildung vorgesehene Zelltypen Epithelzellen (Keratinozyten), Knorpelzellen (Chondrozyten), Fibroblasten oder Endothelzellen Anwendung finden.

Die Transportbedingungen garantieren ein Anwachsen der Zellschichten und die Koloniebildung der Zellen in vivo auf der Wunde. Sie ermöglichen lange Transportzeiten und das Einhalten der gewünschten Innentemperatur von 5 bis 40 ° C und für z.B. Epithelzellen aus Keratinozyten von 20 bis 37°C. Das Transportsystem ist zum Transport biologischer Materialien, die lebende Zellen enthalten, geeignet - z.B. patienteneigenen Materials für autologe, von Fremdmaterialien für allogene Transplantationen wie auch xenogener Materialien.

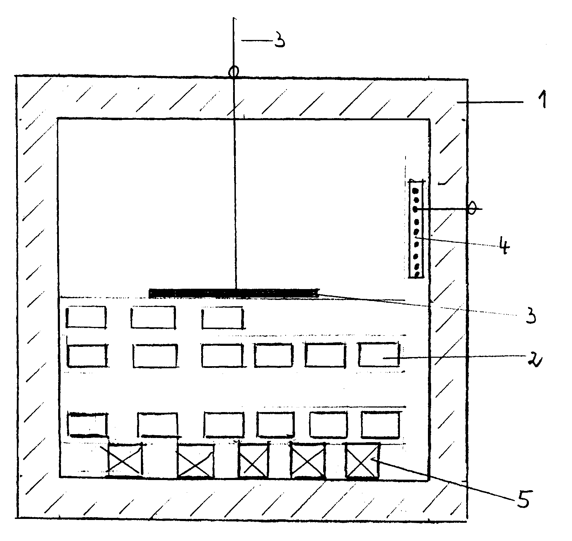

## Patentansprüche

1. Substrat-Zellschichten, enthaltend neue kollagenhaltige oder gelartige Träger sowie Amnion oder azelluläre Dermis als Substrat und eine Schicht aus gezüchteten Zellen.

2. Substrat-Zellschichten nach Anspruch 1, dadurch gekennzeichnet, daß sie die zur Schichtbildung vorgesehenen Zelltypen Epithelzellen (Keratinozyten), Knorpelzellen (Chondrozyten), Fibroblasten oder Endothelzellen enthalten.

3. Substrat-Zellschichten nach Anspruch 1 und 2, dadurch gekennzeichnet, daß sie aus einer kollagenbeschichteten Membran-Keratinozyten-Zellschicht, Amnion-Keratinozyten-Zellschicht, Azellulärdermis-Keratinozyten-Zellschicht, kollagenbeschichteten Membran-Chondrozyten-Zellschicht, kollagenbeschichteten Membran-Fibroblasten-Zellschicht, kollagenbeschichteten Membran-Endothel-Zellschicht, Amnion-Chondrozyten-Zellschicht, Amnion-Fibroblasten-Zellschicht, Amnion-Endothel-Zellschicht, Azellulärdermis-Chondrozyten-Zellschicht, Azellulärdermis-Fibrobasten-Zellschicht, Azellulärderrnis-Endothel-Zellschicht, Gel-Keratinozyten-Zellschicht, Gel-Chondrozyten-Zellschicht, Gel-Fibroblasten-Zellschicht oder einer Gel-Endothel-Zellschicht bestehen.

4. Verfahren zur Herstellung von Substrat-Zellschichten, dadurch gekennzeichnet, daß auf ein Substrat - neue kollagenhaltige oder gelartige Träger sowie Amnion oder azelluläre Dermis - in einem Nährmedium ein die spätere Zellschicht bildende< Zelltyp - Epithelzellen (Keratinozyten), Knorpelzellen (Chondrozyten), Fibroblasten oder Endothelzellen - gebracht, kultiviert und vermehrt werden.

5. Amnionpräparate, bestehend aus Amnien allogener oder xenogener Natur, die aus von der Plazenta abgetrennten Embryonalhüllen nach Entfernen anhaftender Bestandteile und der Chorionanteile hervorgehen.

6. Amnionpräparate nach Anspruch 5, herstellbar dadurch, daß die Embryonalhüllen nach Spontangeburten von der Plazenta abgetrennt, tiefgefroren zwischengelagert, nach der infektionsserologischen Freigabe aufgetaut, von anhaftendem Blut und Gewebe befreit, in feuchtem Zustand das Chorion abgestreift, die verbleibenden Amnien zugeschnitten, sterilisiert, gewaschen, gefriergetrocknet und nach Konservierung bei Raumtemperatur gelagert werden.

7. Verfahren zur Herstellung der Amnionpräparate nach Anspruch 5 und 6, dadurch gekennzeichnet, daß die Embryonalhüllen bei Temperaturen von mindestens -10°C gefroren zwischengelagert, das Abstreifen des Chorions auf einer desinfizierbaren festen Unterlage erfolgt, die Sterilisation mit einem Peressigsäure-Alkoholgemisch, der Waschvorgang mit isotonischer steriler Kochsalzlösung und sterilem Glycerin und die Endverpackung in sterilem Glycerin vorgenommen wird.

8. Verwendung der Zellschichten und der Amnionpräparate nach Anspruch 1 bis 6, dadurch gekennzeichnet, daß sie als Hautersatzmaterial oder als Trägermaterial für mehrschichtige Zellschichten, bei Verbrennungswunden und als Trägermaterial insbesondere für Keratinozyten eingesetzt werden.

9. Transportsystem für Zellschichten und Amnionpräparate, bestehend aus einer bruchsicheren Außenverkleidung und einem Innenraum, der lose Zwischenböden zur Lagerung der Zellschichten enthält - die durch Stempel fixiert werden - und der, neben dem Nährmedium, Heizquellen zur Temperaturregelung und eine steuerbare CO₂-Zufuhr enthält.

10. Transportsystem nach Anspruch 9, dadurch gekennzeichnet, daß es - als Kombination bekannter und neuer Teile - eine Außenverkleidung enthält, die vorzugsweise aus rostfreiem Stahlblech und die Isolierung des Behälters aus Kunststoff, vorzugsweise aus Polystyrol, besteht und als Heizquellen Batterien bzw. aufladbare Akkumulatoren Verwendung finden.
